(19) Europäisches Patentamt
European Patent Office
Office européen des brevets

(11) **EP 4 393 399 A1**

(12) **EUROPEAN PATENT APPLICATION**
published in accordance with Art. 153(4) EPC

(43) Date of publication:
**03.07.2024  Bulletin 2024/27**

(21) Application number: **22864431.6**

(22) Date of filing: **26.08.2022**

(51) International Patent Classification (IPC):
***A61B 5/256*** (2021.01)      ***A61B 5/27*** (2021.01)
***A61B 5/33*** (2021.01)       ***A61B 5/332*** (2021.01)

(52) Cooperative Patent Classification (CPC):
**A61B 5/256; A61B 5/27; A61B 5/33; A61B 5/332**

(86) International application number:
**PCT/JP2022/032215**

(87) International publication number:
**WO 2023/032847 (09.03.2023 Gazette 2023/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **03.09.2021   JP 2021144343**

(71) Applicant: **Xenoma Inc.
Tokyo 143-0013 (JP)**

(72) Inventors:
• **AMANO, Shinichi
Tokyo 143-0013 (JP)**

• **KOBAYASHI, Hirotake
Tokyo 143-0013 (JP)**
• **AMIMORI, Ichiro
Tokyo 143-0013 (JP)**
• **ABE, Natsumi
Tokyo 143-0013 (JP)**

(74) Representative: **Osterhoff, Utz
Bockermann Ksoll
Griepenstroh Osterhoff
Patentanwälte
Bergstraße 159
44791 Bochum (DE)**

(54) **ELECTROCARDIOGRAM EXAMINATION METHOD**

(57)    [Problem] The electrocardiographic testing method with the two or more-lead that the patient can put an electrocardiogram measurement electrode themselves without any special knowledge, hereby it does not need to visit the hospital, and it can also take a bath on the way, as well as it enables an advanced diagnosis, is provided.

[Solution] An electrocardiographic testing method of the two or more-lead using the clothes comprises at least the three measurement electrodes or a measurement electrode attachment part and a connector part for fixing a measurement device,
the clothes have at least two or more different sizes,
the clothes have a neckline encircles the neck, the first measurement electrode or its measurement electrode mounting part is arranged within a circle with a radius of 5cm is centered around a position that 7.5 to 15cm downward from a center point of a straight line connecting to vertices of a left and a right of the neckline,
having the following steps:
(1) a step of obtaining a physique information of a patient;
(2) a step of selecting a size of clothes according to the physique information of the patient;
(3) a step of giving the selected clothes and the measuring device to the patient;
(4) a step of the patient puts on the clothes and the measurement device; and
(5) a step of starting the electrocardiogram measurement;, is characterized.

Fig. 1

START

S1 — Obtain physical information of patient

S2 — Select clothes size

S3 — Give clothes and device to patient

S4 — Patient puts on clothes and device

S5 — Start electrocardiogram measurement

END

## Description

### Technical Field

The present invention relates to a biopotential testing method that can be worn by a patient themselves, particularly an electrocardiographic testing method of a two or more-lead.

### Background Art

A number one cause of a death in worldwide in recent years is ischemic heart disease, accounting for 16% of the all deaths. The ischemic heart disease refers to a disease that causing damage to a heart that a blood flow to a myocardium is obstructed by occlusion or narrowing of a coronary artery, for detecting this disease, it is effective that an electrocardiogram, particularly a 24-hour electrocardiogram using a Holter electrocardiograph. The conventional Holter electrocardiograph are difficult to wear unless a medical worker, it was required that the patient wears at a hospital and takes off at the hospital to 24 hours later again. In addition, since it cannot be removed for a time of a wearing, there is an inconvenience such as not being able to take a bath or the like. For solving this problem, for instance, Non-Patent Literature 1 discloses a service that undergo a 24-hour electrocardiogram medical testing of a lead NASA that the patient receives the Holter electrocardiograph by mail and wears themselves. In the service, an electrocardiogram data is sent to an iPhone by using Bluetooth and a data is sent from the iPhone to a cloud server via Internet. The Patient will not need to go to the hospital, and will be able to remove the device before taking a bath and wear again on after taking the bath.

From a viewpoint of a comfort of the patient, a clothing-type electrocardiograph is also becoming popular. For instance, Non-Patent Literature 2 discloses a case that it was detected that Latent atrial fibrillation by measuring the electrocardiogram using a T-shirt type electrocardiograph for about two months in a young subject under 65 years old. The subject choses either an M or L T-shirt with a measurement electrode placed on a right chest and a left chest, and wore themselves, hereby measuring the electrocardiogram by a lead CC5. The electrocardiogram data measured by a communication device is sent to a smartphone via the Bluetooth, further sending from a smartphone to the data server via 4G network.

Furthermore, Non-Patent Literature 3 discloses the T-shirt type electrocardiograph that an electrode and a wiring that using a conductive silver paste is formed into a film form. The electrocardiogram is measured using the lead CC5 by a measuring electrode on the left chest and the right chest, and a neutral electrode is placed as a ground near a sternum manubrium. Since around the sternum manubrium is less susceptible to an effect of a myoelectric, it seems to be used as the ground. In this literature, it was carried out by keeping a physique of the subject that somewhat similar a height of 170.0 ± 1.4 cm and a weight of 65.8 ± 6.3 kg.

The electrocardiograms in Non-Patent Literature 1, Non-Patent Literature 2, and Non-Patent Literature 3 are a 1-lead, however for instance, in Non-Patent Literature 4 discloses the electrocardiograph of a 6-lead by a monopolar thoracic lead that a stretchable wiring and the measurement electrode are formed inside a commercially available compression shirt. Although it is not specified in these literatures, the subject is one person, and it seems that the physique was not taken into consideration.

Further, Patent Literature 1 discloses, not a shirt-like shape, a 12-lead electrocardiograph is used a band-shaped electrode band that is attached to the chest. Paragraph [0008] states that a minimally trained or untrained people perform an accurate ECG test, and paragraphs [0066] and [0067] state also that the size of the electrode band is determined according to the physique or the like of the patient.

Further, Non-Patent Literature 5 discloses a belt-shaped the 12-lead electrocardiogram measurement made of the textile. It is assumed that it is mainly worn by medical personnel for an emergency lifesaving purpose, however, about a band shaped cloth for placed the measurement electrodes, in alignment with the two landmarks that a midline and an armpit of the patient, by only it is wrapping around the chest of the patient, the measurement electrodes can be placed in the correct position, along to a guideline written on the belt, according to a body size of the patient, by selecting on the button any of S, M, L, or LL on the measuring device, without making a fine adjustment of an electrode position, it is possible to measure the electrocardiogram accurate.

### Prior Art List

### Patent Literature

Patent literature 1: Japanese Unexamined Patent Application Publication No. 2020-163220

**Non Patent Literature**

Non-Patent literature 1: Takami, M.,et al. Circulation Reports (2021): CR-21.

Non-Patent literature 2: Fukuma, N., et al. Scientific reports 9.1 (2019): 1-6.

Non-Patent literature 3: Narihiro Shiozawa, Biomedical engineering, 54.3 (2016):135-138.

Non-Patent literature 4: Jun Hagiwara, et al. Electronics Packaging Academic Conference Collection of Lecture Papers 32nd. Electronics Packaging Academic Conference. The Japan Institute of Electronics Packaging, 2018.

Non-Patent literature 5: https://news.mynavi.jp/article201800117-572956/

**Summary of Invention**

**Technical Problem**

The inventors have investigated that the patient can wear the device themselves, it does not need to visit to the hospital to be dressed by a medical professional, and it can also take a bath and use more comfortable, the Holter electrocardiograph capable of detailed analysis of the two or more-lead, hereby, it has discovered that there were various problems.

One of the problems that the inventor noticed is a positioning work of the measurement electrode based on the physique information. Non-Patent Literature 1 is a small device that does not have a reference position, and it seems to be used by the patient using an illustration or the like. Non-Patent Literature 2 also is the 1-lead, and the measurement electrode position did not need to be exact. Non-Patent Literature 3 is, excepting for there is the neutral electrode near the sternum manubrium, the same as Non-Patent Literature 2 and since the measurement electrode position during a measurement is the 1-lead, a precision did not be much required. About the position of the neutral electrode, when it was located at a position that unrelated to an electrocardiogram measurement lead, there were no restrictions.

Non-Patent Literature 4 is a T-shirt type electrometer of the 6-lead by the monopolar thoracic lead, however the body size is not taken into consideration. When a number of a lead are increasing, although the position of the measurement electrode becomes also an important issue in the electrocardiographic measurement as a medical device, as it is common not only to Non-Patent Literature 4 but also to Non-Patent Literatures 2 and Non-Patent Literatures 3, in an electrocardiographic measurement using the clothes, in this research field, a main theme is noise reduction in the electrocardiogram measurement used a non-adhesive measurement electrode such as the conductive fabric or the like, hereby an accurate measurement electrode positioning is not fully resolved yet.

About the physique and the adjustment of a measurement electrode position, the same arrangement that the measurement electrodes on the chest are placed in Non-Patent literature 4, in Patent Literature 1 of the 12-lead, paragraph [0057] states that the first placement instruction is specified to a sternum part and the second placement instruction is specified to a left nipple. however, in this method, although it is possible to understand whether an above or a below of a nipple by an arrow or the like, an area called the sternum part cannot be easily understood by the patient without a medical knowledge. Furthermore, it is necessary to place the electrodes on a right shoulder, a left shoulder, a left lower abdomen, or the like via cables, however, it cannot place easily without a prior knowledge that placing where part on the shoulder or an abdomen.

Furthermore, as the same, the Non-Patent Literature 5 of the 12-lead discloses using two landmarks on a midline and under an armpit of the patient. Non-patent Literature 5 does not seem to be intended for a non-medical worker, hereby there is no problem, however for supposing when worn by the non-medical worker, hereby it is not easy to wear for the non-medical worker, at first, it is difficult that an operation for measuring a one's own physique using the belt, and it is difficult to align the midline, even if having the medical knowledge, it is so difficult.

The second problem that the inventors noticed was a maintenance. In a testing method by a mail as in Non-Patent Literature 1, it is easy to check the Holter electrocardiograph when returned. Since only a disposable gel electrode come into contact with a body of the patient, there is not a hygiene issue. however, in the case of the electrocardiometry using the clothes, since a sweat and a dirt of the patient adhere to the clothes, it is required a washing step that it is not necessary for the device as in Non-Patent Literature 1. The T-shirt type electrocardiograph of Non-Patent Literature 2 is a commercially available product, and it is assumed that a purchaser owns and washes at home. However, in the case of the electrocardiographic testing for a medical examination and a health checkup, since it becomes the issue that not only the device being measuring but also the clothes that corresponds to the cable deteriorates, an operation confirmation is required more careful. As noted in Non-Patent Literature 2 and Non-Patent Literature 3, the conventional electrocardiogram measurement using the clothes was importance to the comfort of the clothes, and it was importance to consider the ease of having one's own shirt and a psychological hygiene aspect of not wanting to wear the clothes of someone else, and using clothes of someone else, it was seen as a negative factor for the patient.

The present invention has been made in a view of an above circumstance, and the object is realizing that the electrocardiographic testing method can solve the above problems at once. More specifically, the object is providing the electrocardiographic testing method with the two or more-lead that the patient can put an electrocardiogram measurement electrode themselves without any special knowledge, hereby it does not need to visit the hospital, and it can also take a bath on the way, as well as it enables an advanced diagnosis.

**Solution to Problem**

As a result of an intensive study, the present inventors have discovered that based on apparel technology is a field different from an anatomy, by using the clothes with a neckline that encircles the neck, about the sternum manubrium that an anatomical marker position in the electrocardiogram measurement, it is possible to accurately estimate the at area near the neck of the collar. Furthermore, as this area is a first measurement electrode, it was found that a fifth intercostal assumed height could be also easily and accurately estimated from the physique. Non-Patent Literature 3 has the neutral electrode near the sternum manubrium, however since this is not an electrode for the electrocardiographic measurement, it is not required that an accurate position. The present invention differs from a function of the clothes to a conventional technology, and about the placement of the measurement electrode in the two or more-lead that difficult for non-medical worker, by using as a template to fit the clothes to the body physique, a feature of the invention is that it is possible to anatomically accurately specify the sternal manubrium required for the lead NASA and the lead CM5, as well as the fifth intercostal assumed height required for the lead CC5 and the lead CM5.

Furthermore, for it is necessary to guarantee an accuracy for enable an advanced medical diagnosis as an electrocardiogram test method, and it is necessary to wash the clothes that are also the equipment, hereby, without the patient purchases the shirt as the conventional technology is assumed, by having a step that the patient returns both the clothes and the device, the present invention was completed by realizing, a concern of the patient, both a guaranteed operation of the electrocardiogram measurement and wash the clothes.

In order to achieve the above object, one aspect of the present invention provides, in the electrocardiographic testing method of the two or more-lead using the clothes comprises at least the three measurement electrodes or a measurement electrode attachment part and a connector part for fixing a measurement device, wherein, the clothes have at least two or more different sizes, the clothes have a neckline encircles the neck, and vertices of a left and a right of the neckline are connected with a straight line, and the first measurement electrode or its measurement electrode mounting part is arranged within a circle with a radius of 5cm is centered around a position that 7.5 to 15cm downward from a center point of the line,

a testing method characterized by having following steps:

(1) a step of obtaining the physique information of the patient; (2) a step of selecting the size of the clothes according to the physique information of the patient; (3) a step of giving the selected clothes and the measuring device to the patient; (4) a step of the patient puts on the clothes and the measurement device; and (5) a step of starting the electrocardiogram measurement.

According to a one aspect of the present invention, the even patient without the medical knowledge, by taking advantage of a characteristics of an apparel, it can easily place the measurement electrode accurately at the sternal manubrium that is an anatomical marker positionand, it can perform the electrocardiogram of the two or more-lead themselves. it becomes possible to perform the lead NASA and the lead CM5 that a typical lead that the sternum manubrium is the measurement electrode, in addition lead CM1, CM2, CM3 or the like.

As another aspect of the present invention, after the above-mentioned a step of (5) is completed, it is preferable that the electrocardiogram testing method is characterized by having following a series of steps:

(6) a step of the patient returns the clothes and the devices;

(7) a step of reporting the electrocardiogram testing results to the patient;

(8) a step of washing the clothes; and

(9) a step of checking the operation of the clothes and the devices.

Hereby, it is possible that an electrocardiogram test service provider performs maintenance easily, and a reliability of the electrocardiogram test is improved.

As further other aspect of the present invention, it is preferable that the electrocardiographic testing method is characterized in the physique information includes the height. In the physique information, the height is effective for estimating a distance between the sternum manubrium and the fifth intercostal assumed height.

As further other aspect of the present invention, when the height is H and the vertical distance between the first measurement electrode and the fifth intercostal assumed height is D, it is preferable that the electrocardiographic testing method is characterized in H and D satisfy the relationship of the following formula [A]. By this, it estimates the distance between the sternum manubrium and the fifth intercostal assumed height.

(Math. 4)

$$D = [(H - 119.390) / 2.47] \pm 5.0 \ [A]$$

As further other aspect of the present invention, when the height is H and the vertical distance between the first measurement electrode and the fifth intercostal assumed height is D, it is preferable that the electrocardiographic testing method is characterized in H and D satisfy the following formula [B] when the patient is a male, and the following formula [C] when the patient is a female. By this, it estimates the distance between the sternum manubrium and the fifth intercostal assumed height.

(Math. 5)

$$D = [(H - 126.388) / 2.23] \pm 5.0 \ [B]$$

(Math. 6)

$$D = [(H - 110.713) / 2.76] \pm 5.0 \ [C]$$

As further other aspect of the present invention, it is preferable that the electrocardiographic testing method is characterized in the physique information further includes at least one of a body weight, a chest circumference, and an abdominal circumference. By this, it estimates the width direction of the physique, and it is possible to become to estimate accurately where placing the positive measurement electrode on the line of the fifth intercostal assumed height, further for instance, it is possible to become to distinguish the lead CM1, the lead CM2, the lead CM3, and the lead CM5.

As further other aspect of the present invention, it is preferable that the electrocardiographic testing method is characterized in including the three leads of the lead NASA, the lead CM5, and the lead CC5.

As further other aspect of the present invention, it is preferable that the electrocardiographic testing method is characterized in the step (5) of starting the electrocardiogram measurement includes a step of informing to the patient whether the clothes and the measurement device are correctly put.

As further other aspect of the present invention, it is preferable that a step of informing to the patient whether correctly put is characterized in at least one of a vibration, a light, and a sound from the measurement device or a terminal that wirelessly communicates with the measurement device. It is also possible to be decided by showing the electrocardiogram waveform to the patient, however about the information automatically determined by a system, by informing thought a simple method such as the vibration, the light, or the sound from the terminal, it becomes a better safer service for the patient.

As further other aspect of the present invention, it is preferable that electrocardiogram testing method is characterized in an obtained data is transmitted and recorded to a server directly from the measurement device or via the terminal wirelessly communicates with the measurement device. Even if the measuring device is lost during a delivery step between the patient and the service provider, there is no risk of leaking the electrocardiogram data that is a personal information of the patient.

As further other aspect of the present invention, it is also preferable that the electrocardiographic testing method is characterized in the data obtained by the electrocardiographic measurement is recorded on a recording medium within a device. About a transmitting data by the wireless communication, there is a concern that the data may be lost by a communication environment or the like, furthermore, since the data size of a multi-lead electrocardiogram data is large, a communication cost may be high. For a leakage of the electrocardiographic data by a theft or the like of the measuring device, it is preferable to encrypt a recorded data.

As further other aspect of the present invention, it is preferable that the electrocardiographic testing method is characterized in the measuring device or a terminal that wirelessly communicates with the measuring device includes means for recording a predetermined event.

As further other aspect of the present invention, it is preferable that the electrocardiographic testing method is characterized in it is also recorded an exercise data from at least one of an acceleration sensor and a gyro sensor.

As further other aspect of the present invention, it is preferable that the electrocardiographic testing method is characterized in a stretchable wiring includes a core material with stretchability;

the stretchable wiring having: a conductive covering part that about a conductive fiber that at least one fiber is coated with a conductive material, via directly or through other materials, wrapped around and covering the core material; and an insulating covering part that an around of the conductive covering part that is covered with an insulating material; when the length of the stretchable wiring is L1 and the length after removing the insulating covering part from the stretchable wire is L2, 0.50<L2/L1<1.00. This makes it is possible to suppress that increasing a resistance caused by an insulating layer during stretching of the stretchable wiring.

The method for producing the insulating covering part is as follows, a method of injection molding directly an elastomer such as a natural rubber or a synthetic rubber to around of the conductive covering part, a method of dipping the conductive covering part in a solution of the elastomer, or a method of wrapping the insulating fiber to a spiral manner or a braided manner or the like, can be mentioned. the synthetic rubber includes a polystyrene-based, a polyvinyl chloride-based, a polyurethane-based, a polyester-based, a polyamide-based, an olefin-based, a silicone-based, and a fluorine-based, or the like can be mentioned. The insulating fiber are not particularly limited as long as an insulating, and includes a natural fiber, a polyester fiber, a polyamide fiber, a polyurethane fiber, a polyacrylic fiber, a polyolefin fiber, or the like, can be mentioned. It is preferable that the insulating fiber is coated a water repellent coat in advance in order to improve an insulation property. The water repellent coat is not particularly limited, and includes the fluorine-based and the silicone-based or the like, can be mentioned.

As further other aspect of the present invention, it is preferable that the electrocardiographic testing method is characterized in about the wiring that connecting the measurement electrode or the measurement electrode attachment part and the connector part, when an extension is extended by 20% from the original wiring length, an electrical resistivity increases from 0 to 30%. In order to realize both a comfort for the patient and a precision, it is preferable to use the optimal stretchable wiring about a resistance stability during a stretching. And the electrical resistivity increase of the stretchable wiring is, about a 40cm long wiring, determined by measuring the resistance value of both ends using a tester, an initial resistance value was R0, next adjusting that so as to the wiring became a 48cm and a 20% extended, a resistance value of both ends was measured using a tester again, the resistance value of the 20% extended was set as R1, and the electrical resistivity increase r (%) = (R1-R0)/R0 x 100 was calculated.

As further other aspect of the present invention, it is preferable that about the electrocardiographic testing method is characterized in about a fabric used in a range of 10 cm downward and 5 cm wide from the first measurement electrode or the measurement electrode attachment part, when the vertical direction 4.9N is loaded, an extension rate is 160% or less. A compression shirt made of a stretchable fabric were the mainstream that was the clothes using by the conventional electrocardiogram measurement, however, about the multi-lead electrocardiogram of the present invention that has the first measurement electrode on the sternal manubrium, in order to reduce a distance change between the sternal manubrium and the fifth intercostal assumed height, it is preferable that the fabric below the first measurement electrode does not have a great elasticity.

As further other aspect of the present invention, it is preferable that the electrocardiographic testing method comprises: a mechanism that about a part of the clothes including a portion that the measurement electrode or the measurement electrode attachment part is attached, it comprises a mechanism can be detached in a whole or in the part from the clothes.

**Brief Description of Drawings**

FIG. 1 is a flowchart showing a first embodiment of an electrocardiographic testing method according to the present invention.

FIG. 2 is a diagram showing an appearance of a clothes 1 used in an electrocardiographic testing method shown in FIG. 1.

FIG. 3 is a flowchart showing a second embodiment of an electrocardiographic testing method according to the present invention.

FIG. 4 is a diagram showing a fifth intercostal assumed height of clothes 1 used in an electrocardiographic testing method according to the present invention.

FIG. 5 is a diagram related to a lead NASA of an electrocardiogram measurement according to the present invention.

FIG. 6 is a diagram regarding a lead CM5 of an electrocardiogram measurement according to the present invention.

FIG. 7 is a diagram regarding a lead CC5 of an electrocardiogram measurement according to the present invention.

FIG. 8 is a diagram showing an appearance of clothes 1 of another embodiment used in an electrocardiographic testing method according to the present invention.

FIG. 9 is a flowchart showing a third embodiment of an electrocardiographic testing method according to the present invention.

FIG. 10 is a block diagram of a measurement device 31 according to the present invention.

FIG. 11 is a diagram showing an appearance of clothes 1 of another embodiment used in an electrocardiographic testing method according to the present invention.

FIG. 12 is a diagram showing an appearance of clothes 1 of another embodiment used in an electrocardiographic testing method according to the present invention.

**Description of Embodiments**

Hereinafter, the preferred embodiments of the present invention will be described in accordance with accompanying drawings.

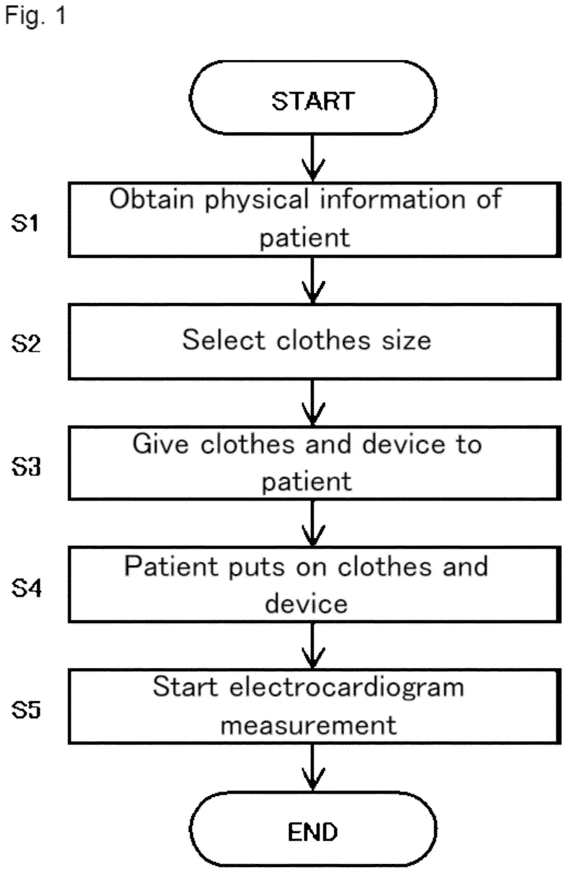

FIG. 1 is a flowchart of an electrocardiographic testing method according to the present invention. For instance, when the present invention is used for a service such as a health checkup, by a patient fill out an application form or the like, a testing service provider obtains a physical information such as a height and a weight of the patient in advance (step S1).

Next, based on the physique information, so as to the measurement electrode are placed at an appropriate position according to the physique of the patient, the testing service provider selects a size of clothes for an electrocardiogram measurement (step S2).

Next, the testing service provider sends (hands over) the selected clothes and a measurement device to the patient (step S3). The sending may be delivered by a hand to the patient, or a mail or a home delivery service or the like, and from a viewpoint of a convenience for the patient, it is preferable to send to a home of the patient by the mail or the home delivery service. In addition, a sending product may also include an explanatory manual, and, if necessary, it may also include a disposable gel electrode, and, for the changing clothes, the additional clothes for the electrocardiogram measurement.

Next, the patient wears on the clothes themselves and attaches the measuring device to a connector part (step S4).

Next, the patient starts the electrocardiogram measurement (step S5). The electrocardiogram measurement may be started automatically by confirming the connector part is energized when the measurement device is attached to the connector part, or it may be started intentionally by the patient turning on a switch or the like. Alternatively, it may be paired with an information processing terminal such as a smartphone using Bluetooth or the like, and it may be started using a smartphone application installed in the information processing terminal. In this case, when the patient does not have the information processing terminal such as the smartphone or a tablet terminal, it causes a disadvantage that the electrocardiogram measurement cannot be performed, hereby it is preferable that an electrocardiogram can be measured without the information processing terminal such as the smartphone.

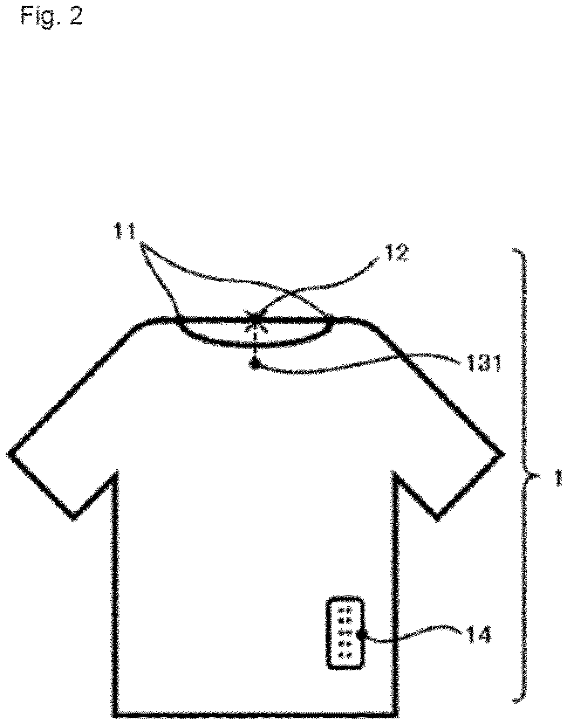

FIG. 2 shows an appearance of the clothes 1 used in the electrocardiographic testing method shown in FIG. 1 according to the present invention. Since the clothes 1 is worn directly on a bare skin, it is preferably a short-sleeved T-shirt type or a sleeveless type that is easy to wear as an underwear regardless of a season. The clothes have a neckline that passes through the neck, and occurring when a front side and a back side are contacted together, vertices 11 of a left and a right of the neckline are connected with a straight line, and within a circle with a radius of 5 cm centered at a position 7.5 to 15 cm downward from a center point 12 of a line segment, a first measurement electrode or measurement electrode mounting portion 131 is arranged. A device for measuring the electrocardiogram is attached to the connector section 14.

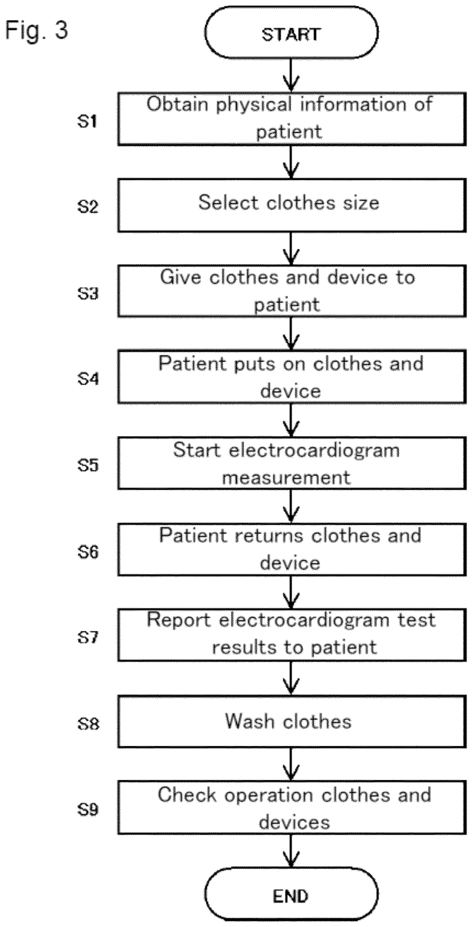

FIG. 3 is the flowchart showing a second embodiment of an electrocardiographic testing method according to the present invention. After the electrocardiogram measurement is started in step S5 of FIG. 1, after the measurement is completed, the patient takes off the device and the clothes in addition returns to the service provider (step S6). Since this will reduce a burden of the patient, when sending in step S3, it is preferable to send to package together that a return packaging material and a return address document or the like.

Next, the service provider analyzes an electrocardiographic data of the patient and outputs the electrocardiographic test results, in addition reports to the patient (step S7). The results may be an electronic data such as PDF, or may be printed on a paper.

Next, the service provider washes the clothes already worn by the patient (step S8). Washing may be using a washing machine for a home or a commercial washing machine, and it may be a cold washing or a dry washing. From a hygiene perspective, it is preferable that high-temperature washing in hot water of 80°C or higher for a sterilization, however, since a 24-hour electrocardiogram are generally performed by the healthy patient that enough to carry out daily activities, it is preferable that a normal water washing from the viewpoint of a damage to the clothes.

Next, the service provider checks the operation of the clothes and the measuring device (step S9). This step does not need to be performed every time for the all devices according to an operating method, however the measurement device always needs to be charged. Furthermore, since there is a possibility that there is a damage in the wiring of the clothes in the above step S8, it is preferable that checking for whether there is not a breakage in the wiring and measuring an electrical resistance of both ends of the measurement electrode or a measurement electrode attachment part and the connector part.

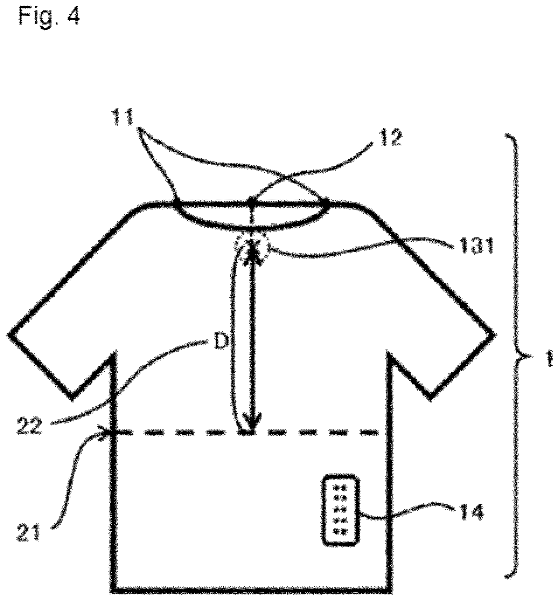

FIG. 4 shows, based on a fifth intercostal assumed height 21 of the clothes 1 used in the electrocardiographic testing method according to the present invention, a distance D between the first measurement electrode or a measurement electrode attachment part 131 and the fifth intercostal assumed height. The following relational expression exists between the distance D indicated by a reference numeral 22 in FIG. 4 and a height H of the patient.

(Math. 7)

(In case of there is no a sex information)

$$D = [(H - 119.390) / 2.47] \pm 5.0 \ [A]$$

( Math. 8)

(In case of the male)

$$D = [(H - 126.388) / 2.23] \pm 5.0 \ [B]$$

( Math. 9)

(In case of the female)

$$D = [(H - 110.713) / 2.76] \pm 5.0 \ [C]$$

About the distance between the sternal manubrium of a human body and the fifth intercostal assumed height, a high correlation between the height and a sternum length has been shown in, for instance, Yonguc, G. N. , et al. "Estimation of stature and sex from sternal lengths: an autopsy study." Anatomical science international 90.2 (2015): 89-96. A sternum consists of the sternum manubrium, a sternum body, and a xiphoid process, and D corresponds to a length of a sum of the sternum manubrium and the sternum body, table 2 shows the above formula [B] for the male and the above formula [C] for the female. In the present invention, when a sex is not taken into consideration, it is used that the above formula [A] that is an average of the formula [B] and the formula [C], and it has been found that it still shows sufficient accuracy.

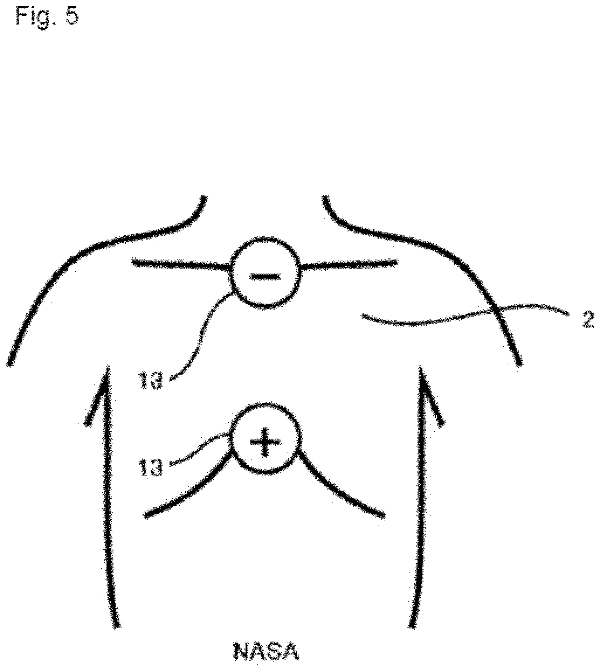

FIG. 5 is a diagram regarding the lead NASA of the electrocardiogram measurement according to the present invention.

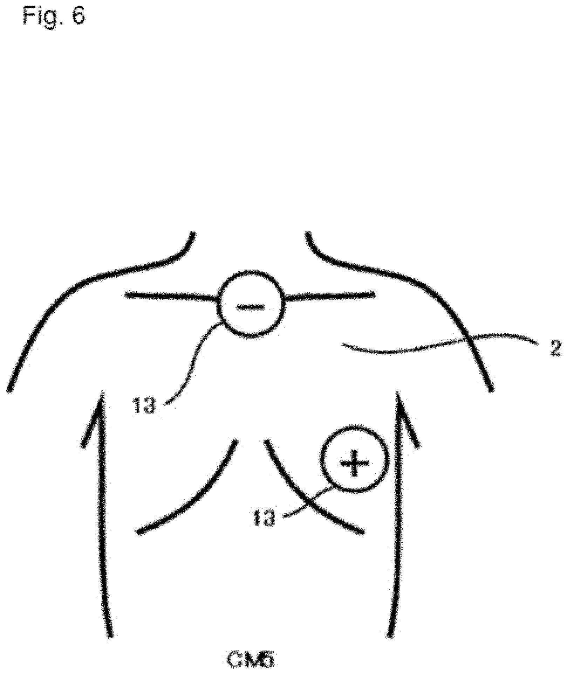

FIG. 6 is the diagram regarding the CM5 lead of the electrocardiogram measurement according to the present invention.

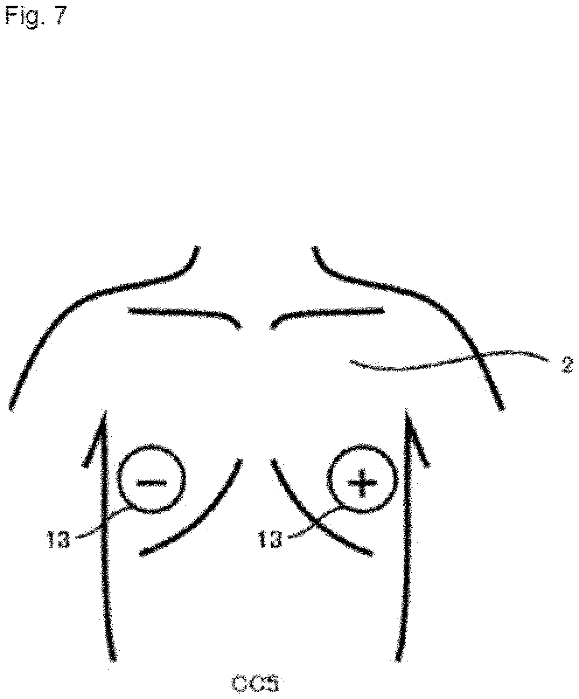

FIG. 7 is the diagram regarding the lead CC5 of the electrocardiogram measurement according to the present invention.

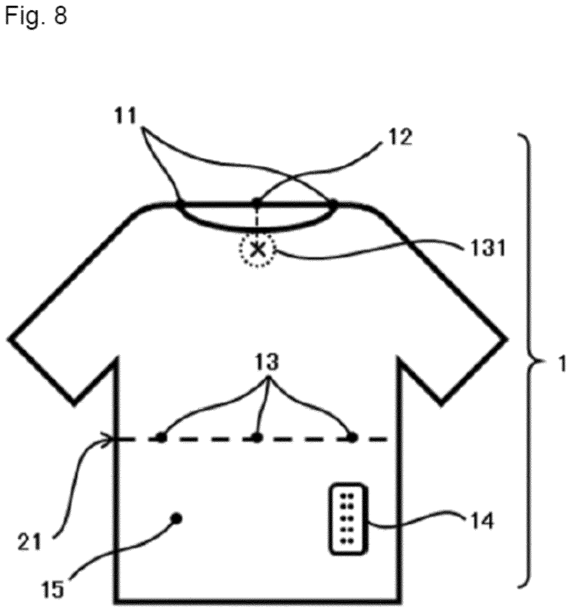

FIG. 8 is the diagram showing an appearance of another embodiment of the clothes 1 used in the electrocardiographic testing method according to the present invention. Three measurement electrodes 13 are placed on a virtual line of the fifth intercostal assumed height 21, and it is called an EASI-lead electrocardiogram that the electrocardiogram measurement is using by four electrodes including that the lead NASA, the lead CM5, and the lead CC5, in addition the first measurement electrode, and as an alternative to the most detailed 12-lead electrocardiogram, for instance, "Deriving the 12-lead electrocardiogram from four (EASI) electrodes." by Dower et al. Journal of electrocardiology 21 (1988): S182-S187, this method has been known for a long time. As an advantage of the EASI- lead electrocardiogram, for instance, as shown in Finlay et al.'s "Effects of electrode placement errors in the EASI-derived 12-lead electrocardiogram." Journal of electrocardiology 43.6 (2010): 606-611, it is known that it is allowed that an error of $\pm 5$ cm in an up down left right from an ideal measurement electrode position. The inventors, by the apparel pattern technology, in order to, only wearing the clothes, an electrocardiogram measurement electrode be able to place in an accuracy of within $\pm 5$ cm relative to this anatomical marker position, it has been found that it is effective to determine the position of the first electrode 131 (measurement electrode) with reference to the vertex 11 of the left side and the right side of a neckline.

Furthermore, it is preferable to arrange a neutral electrode 15 on the clothes 1 in order to reduce an electrocardiogram noise and stabilize a baseline. A place of the neutral electrode 15 is not particularly limited as long as a position that not effected to the measurement, however in the case of shirts, since it is limited to the upper body, it is preferable a wrist or a right-side abdomen, and since it can be a short sleeve, it is particularly preferable the right-side abdomen.

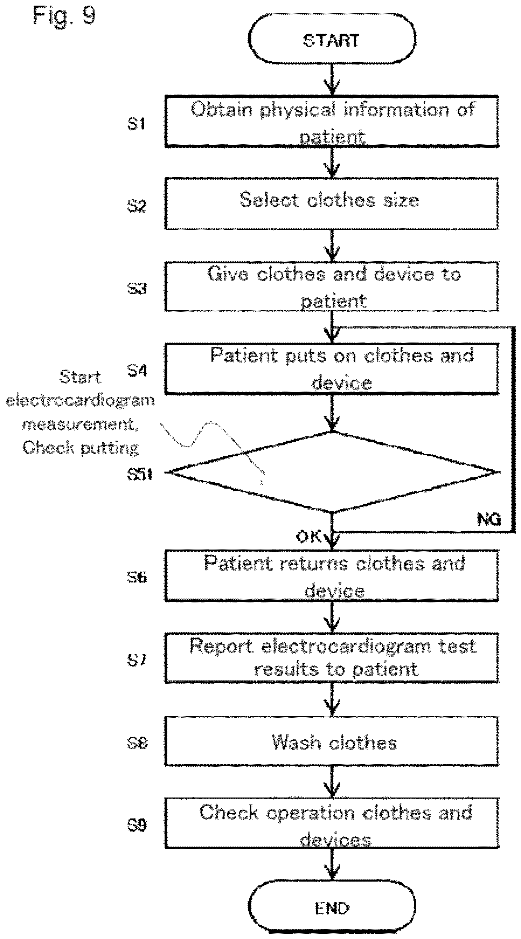

FIG. 9 is the flowchart showing the third embodiment of the electrocardiographic testing method according to the present invention. Instead of step S5 in FIG. 3, after the start of a measurement, based on the electrocardiogram data obtained, it is checked whether the measurement electrodes are correctly put, and if not, the patient is informed and the patient re-puts the clothes. There are no particular restrictions on the method of a notification, however from a viewpoint of a convenience and an intuitive user interface, it is preferable to notify to the patient using a method such as a vibration, a light, or a sound of the device.

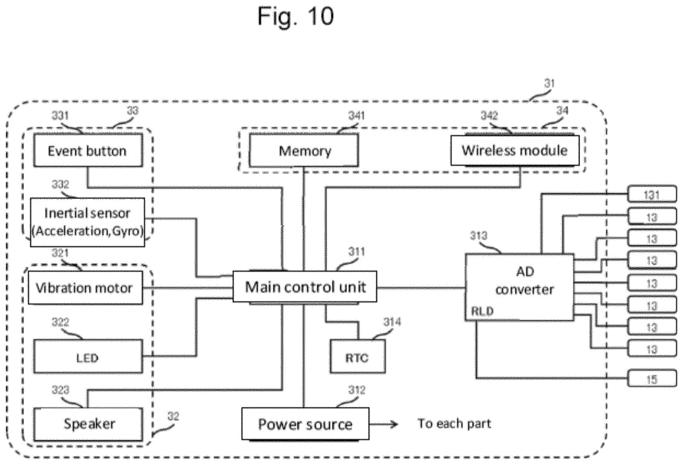

FIG. 10 is a block diagram of the measurement device 31 according to the present invention. Mainly the main control unit 311, a measurement, an event, or an input, or the like are performed. An event is when a patient feels an abnormal in a physical condition during a 24-hour electrocardiogram measurement, for instance, it includes a palpitation, a shortness of a breath, a dizziness, a heart pain, or the like. This makes it possible to analyze a relationship between an abnormality felt by the patient and an electrocardiogram at that time. A power source 312 supplies power not only to the main control part but also to each part. An AD converter 313 is for digitally converting a potential between a pair of the measurement electrodes, and it is preferably to use an amplifier for amplifying the potential. It is preferably that a sampling frequency for the electrocardiogram measurement is 100 Hz or more, and more preferably 250 Hz or more, it is preferably that particularly preferably 500 Hz or more, and most preferably 1000 Hz or more.

Recently, the AD converter for measuring a biopotential such as an electrocardiogram or the like have also appeared. About many of these AD converters, it is existing and preferably that many of these AD converters include a circuit called a RLD (Right Leg Drive) that for connecting a neutral electrode as a ground for removing a noise from a household power source or a radio wave or the like. About the RLD, for instance, Winter et al., "Driven-right-leg circuit design." IEEE Transactions on Biomedical Engineering 1 (1983): 62-66. is shown. About the neutral electrode, even if it is not an RLD, it is not particular limited as long as it is designed to determine the ground of a biological potential on the circuit within the device.

In recent years, by using the electrocardiogram measurement, for monitoring an autonomic nervous system such as a stress, it has been used that a technique called Heart Rate Variability (HRV) that measures a fluctuation of the time interval of a R wave. Although a time accuracy is important in this method, since a clock built into a commercially available microcontroller is easily effected by an environmental temperature, it is preferable that about the measurement device 31, a real-time clock (RTC) 314 that more accurate is used.

In the measurement device 31 according to the present invention, in order to inform to the patient whether the measurement electrode is correctly put as described above, it is preferable to include an information output part 32. About an information output part, it is more preferable to use the vibration from a vibration motor 321, the light from a LED 322, and the sound from a speaker 323. There are no particular limited how to determine whether the electrode are correctly put, however, since for instance, when one or both of a pair of the measurement electrodes is removed, only a large noise signal will be produced, hereby it is preferable to be a method of judge by measuring and detecting a S/N ratio in a certain section, in addition, a method of detecting a heartbeat cycle by Fourier transform, or a method of detecting by using a low-pass filter that emphasizes the R wave of the electrocardiogram. The information output part 32 outputs at least one of the vibration, the light, and the sound when it is determined the measurement electrode is correctly put by the above method, or when it is determined that the measurement electrode is not correctly put.

Furthermore, it is preferable that the measuring device 31 according to the present invention includes an information input part 33 for recording a predetermined subjective symptom and recording an action. About the information input part 33, it is more preferable that there is an event button 331 that the patient actively inputs, and it is particularly preferable that an exercise information of the patient is automatically recorded by an inertial sensor 332 without the patient being aware. In this way, an electrocardiogram measuring device 31 or the terminal that wirelessly communicates with the electrocardiographic measuring device 31 may include means for recording the predetermined event.

Further, the measurement device 31 according to the present invention includes a recording part 34 (a recording medium) for recording not only the electrocardiogram but also the event and the information (the data) of the inertial sensor. It is preferable to use a memory 341 for recording the data. Alternatively, since it is possible to transmit the information to an external device such as the smartphone, a PC, or the Internet via a wireless module 342, or directly, it is preferable. About the data obtained by the electrocardiogram measurement, directly or via the terminal that wirelessly communicates with the electrocardiogram measurement device, it may be transmitted from an electrocardiogram measurement device 31 to the server and recorded. In the case of a 24-hour electrocardiogram, since, by the patient travels, it is possibility that it may not have an Internet environment or it may not carry a smartphone, it is more preferable to record the electrocardiogram in the memory 341 within the measurement device 31, When the clothes 1 includes at least one of an acceleration sensor and a gyro sensor, the exercise data obtained by at least one of the acceleration sensor and the gyro sensor may be recorded together.

About the electrodes (the measuring electrode, the neutral electrode) used for the electrocardiogram measurement according to the present invention, it can be used that a general gel electrode, in addition to a rubber electrode made from a conductive rubber or a fabric electrode made from a conductive fabric. Since it is purpose that the electrocardiogram measurement of the present invention is a high-precision measurement for a medical, it is preferable to use a sticky gel electrode that is not easily effected by a body movement such as a walking. In the case of the gel electrode, since it is generally that a disposable that an attached with a snap button, for attaching the gel electrodes, an electrode attachment part 13, an electrode attachment part 15, and the electrode attachment part 131 are arranged on the clothes 1, however in the case of the rubber electrode or the fabric electrode, a method of arranging by a sewing or a pasting, in addition to, it is also possible to arrange the electrodes 13, 15, and 131 on the clothes 1 by incorporating directly into the fabric by a knitting or a weaving.

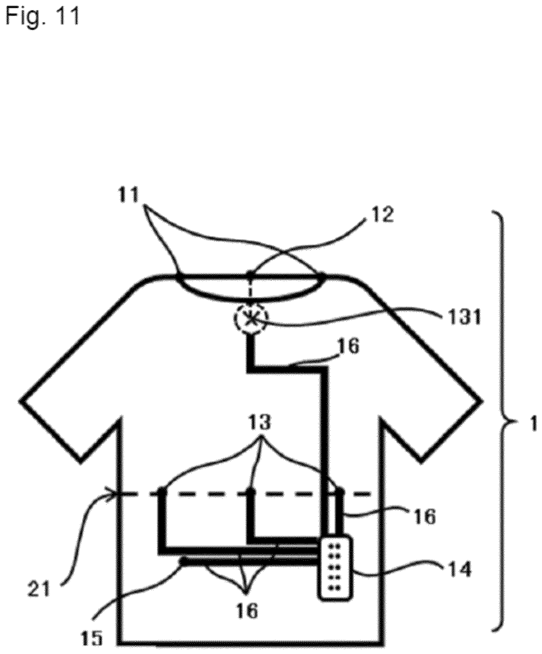

FIG. 11 shows the appearance of the clothes 1 of other embodiment used in the electrocardiographic testing method according to the present invention. The electrodes or the electrode attachment part 13, 15, and 131 are connected to a connector part 14 for fixing a measuring device by a stretchable wiring 16. The stretchable wiring 16 includes a core material with stretchability so as to the resistance does not change by a human movement, including: a conductive covering part coated that about the conductive fiber that at least one fiber is coated by a conductive material, via directly or through another material, coated by wrapped around the core material; an insulating covering part that the around of the conductive covering part that is coated by an insulating material; and in case of a length of the stretchable wire is L1, and the length after the insulating coating part is removed from the stretchable wire is L2, it is preferable that $0.50 < L2/L1 < 1.00$. By this, it is not only reducing a resistance change when the stretchable wiring expands and contracts by a movement or the like of the patient, but also becoming to prevent from the wiring is a breaking by an electrocardiogram measurement shirt is pulled strongly when worn or washed by the patient.

Furthermore, about the clothes 1 used in the electrocardiographic testing method according to the present invention, the first measuring electrode or the measuring electrode attachment part 131 is a reference position of the origin, hereby, in order to avoid the distance D from the fifth intercostal assumed height 21 changes greatly, it is preferable that about the fabric used in an area of a 10 cm downward from the first measurement electrode or the measurement electrode attachment part and a 5 cm width, when the vertical direction 4.9N is loaded, an elongation rate is 160% or less in the vertical direction. In addition, about the elongation rate of the used fabric, based on Method D of "JIS L 1096 Fabric Testing Methods for Woven and Knitted Fabrics", prepare a test piece with a width of 50 mm and a length of 300 mm, mark at 100 mm intervals (L0) and applying a load of 4.9N, it measures the length between the marks (L1) after holding for 1 minute, hereby the elongation rate (%) can be calculated as $\{(L1-L0)/L0\} \times 100$.

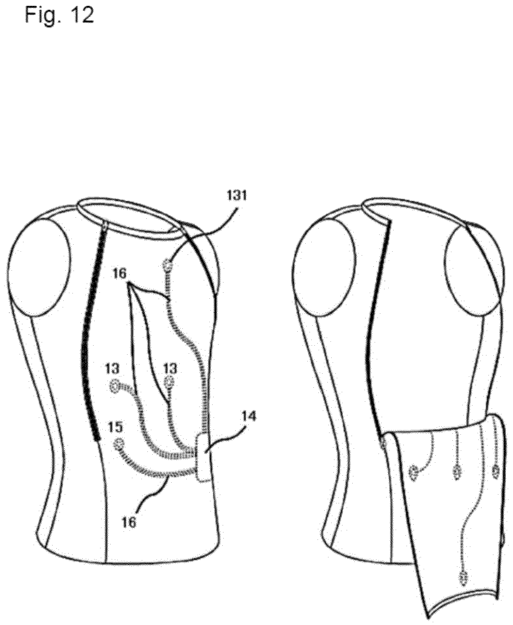

FIG. 12 shows the appearance of clothes 1 of other embodiment used in the electrocardiographic testing method according to the present invention. In case of the clothes 1 is worn over the top, it will be difficult to adjust the position of the measurement electrode or the measurement electrode attachment parts 13 and 131, and, in case of a Y-shirt type or a polo shirt type that fastens with a button in the front, the measurement electrode or the measurement electrode attachment part 131 will be contacted to the button, hereby it is preferable that a part of the clothes 1 is provided with a mechanism that can be removed completely or partially. There are no particular limited on the method for fixing a front opening, however it is preferred that a button, Velcro, or a zipper, and it is more preferred that the zipper from the viewpoint of a convenience and the comfort.

Table 1 shows, as the electrocardiogram measurement shirt of the present invention, when it manufactures in five sizes, XS, S, M, L, and XL, an actual distance of D, the recommended height for the patient for each sex.

[Table 1]

| Size | D (cm) | Sex | Estimated height (cm) | |
|---|---|---|---|---|
| | | | Lower limit | Upper limit |
| XS | 12 | Male | 150 | 160 |
| | | Female | 147 | 160 |
| S | 14 | Male | 155 | 165 |
| | | Female | 150 | 165 |
| M | 16 | Male | 160 | 175 |
| | | Female | 160 | 175 |
| L | 18 | Male | 170 | 185 |
| | | Female | 170 | 185 |
| XL | 19 | Male | 175 | 190 |
| | | Female | 170 | 190 |

For the electrocardiogram measurement shirt in Table 1, for patients between 147 cm and 190 cm in height, applying the formula [A] for a unisex, the formula [B] for a male, and the formula [C] for a female, and table 2 shows the results of calculating the upper and lower limits of D and verifying the correspondence with the recommend size. About a size corresponding to the range of D obtained from the formula, it includes that the size obtained from the recommend size chart based on the height of the patient, and it was confirmed that the electrocardiogram could be measured without any problems.

[Table 2]

| | Lower limit | Upper limit | Formula I Formula adapted size I | Recommend Male | Recommend Female | Lower limit | Upper limit | Formula II Formula adapted size | Recom-mend Male | Lower limit | Upper limit | Formula III Formula adapted size | Recom-mend Female |
|---|---|---|---|---|---|---|---|---|---|---|---|---|---|
| 147 | 5.6 | 156 | XS. S | - | XS | 4.7 | 14.7 | XS. S | - | 6.6 | 16.6 | XS. S. M | XS |
| 148 | 5.9 | 159 | XS. S | - | XS | 4.9 | 14.9 | XS. S | - | 6.8 | 16.8 | XS. S. M | XS |
| 149 | 6.1 | 16.1 | XS. S. M | - | XS | 5.2 | 15.2 | XS.S | - | 70 | 17.0 | XS. S. M | XS |
| 150 | 6.3 | 16.2 | XS. S. M | XS | XS. S | 5.5 | 15.5 | XS. S | XS | 72 | 17.2 | XS. S, M | XS. S |
| 151 | 6.6 | 16.8 | XS S. M | XS | XS. S | 5.8 | 15.8 | XS. S | XS | 7.4 | 17.4 | XS. S. M | XS. S |
| 152 | 6.8 | 168 | XS. S. M | XS | XS. S | 6.0 | 16.0 | XS. S. M | XS | 7.7 | 17.7 | XS. S. M | XS. S |
| 153 | 71 | 17.1 | XS. S. M | XS | XS, S | 6.3 | 16.3 | XS. S. M | XS | 7.9 | 17.9 | XS. S. M | XS. S |
| 154 | 7.2 | 17.2 | XS. S. M | XS | XS. S | 6.6 | 16.6 | XS. S. M | XS | 8.1 | 18.1 | XS. S. M. L | XS. S |
| 155 | 7.6 | 17.6 | XS. S. M | XS. S | XS. S | 6.2 | 168 | XS. S. M | XS. S | 8.3 | 18.3 | XS. S. M. L | XS. S |
| 156 | 7.8 | 17.8 | XS. S.M | XS. S | XS. S | 7.1 | 17.1 | XS. S M | XS. S | 8.6 | 18.6 | XS. S. M. L. | XS. S. |
| 157 | 8.1 | 18.1 | XS. S. M. L | XS. S | XS. S | 7.4 | 17.4 | XS. S. M | XS. S | 8.8 | 18.8 | XS. S. M. L | xs. S |
| 158 | 8.3 | 18.3 | XS. S. M. L | XS. S | XS. S | 7.6 | 17.6 | XS. S. M | XS. S | 90 | 19.0 | XS. S. M. L XL | XS. S |
| 159 | 8.6 | 18.6 | XS. S. M. L | XS. S | XS. S | 7.9 | 17.9 | XS. S. M | XS. S | 9.2 | 19.2 | XS. S. M. L. XL | XS. S |
| 160 | 8.8 | 18.8 | XS. S. M. L | XS. S. M | XS. S. M | 8.2 | 18.2 | XS. S. M. L | XS. S. M | 9.4 | 19.4 | XS. S. M. L. XL | XS S. M |
| 161 | 9.1 | 19.1 | XS. S. b1. E. XL | S. M | S, M | 8.5 | 18.5 | XS. S. M. L | S. M | 9.7 | 19.7 | XS S. M. L XL | S.M |
| 162 | 9.2 | 19.3 | XS. S. M. L. XL | S. M | S. M | 8.7 | 18.7 | XS. S M. L | S. M | 9.9 | 19.9 | XS. S. M. L XL | S. M |
| 163 | 9.5 | 195 | XS. S.M. L. XL | S. M | S. M | 9.0 | 19.0 | XS. S. M. L. XL | S. M | 10.1 | 20.1 | XS. S. M. L XL | S, M |
| 164 | 9.8 | 19.8 | XS. S. M. L. XL | S. M | S M | 9.3 | 19.3 | XS. S. M. L. XL | S. M | 10.3 | 20.3 | XS. S. M. L XL | S. M |

(continued)

| | Lower limit | Upper limit | Formula I Formula adapted size I | Recommend Male | Recommend Female | Lower limit | Upper limit | Formula II Formula adapted size | Recom-mend Male | Lower limit | Upper limit | Formula III Formula adapted size | Recom-mend Female |
|---|---|---|---|---|---|---|---|---|---|---|---|---|---|
| 165 | 10.0 | 200 | XS. S. M. L. XL | S. M | S. M | 9.5 | 19.5 | XS. S. M. L. XL | S. M | 106 | 20.6 | XS. S. M. L XL | S. M |
| 166 | 10.3 | 20.3 | XS. S. M. L. XL | M | M | 9.8 | 19.8 | XS. S. M. L. XL | M | 10.8 | 208 | XS S. M. L XL | M |
| 167 | 10.5 | 20.5 | XS. S. M. i. XL | M | M | 10.1 | 20.1 | XS. S. M. L. XL | M | 110 | 21.0 | XS. S. M. L XL | M |
| 168 | 10.8 | 20.8 | XS. S. M. L. XL | M | M | 10.4 | 20.4 | XS. S M L XL | M | 11.2 | 212 | XS. S. M. L XL | M |
| 169 | 11.0 | 21.0 | XS. S. M. L. XL | M | M | 10.6 | 20.6 | XS. S. M. L. XL | M | 11.5 | 21.5 | XS. S. M. L XL | M |
| 170 | 11.3 | 213 | XS. S. M. L. XL | M. L | M, L XL | 10.9 | 20.9 | XS. & M. L. XL | M. L | 11.7 | 21.7 | XS. S. M. L XL | M. L XL |
| 171 | 11.5 | 215 | XS. S. M. L. XL | M. L | M. L. XL | 11.2 | 21.2 | XS. & M. L. XL | M. L | 11.9 | 21.9 | XS. S. M. L XL | M. L XL |
| 172 | 11.8 | 21.8 | XS. S. M. L. XL | M. L | M. L. XL | 11.4 | 21.4 | XS. S. M. L. XL | M. L | 12.1 | 22.1 | S. M. L. XL | M. L XL |
| 173 | 12.0 | 22.0 | XS. S. M. L. XL | M. L | M. L. XL | 11.7 | 21.7 | XS. & M. L. XL | M. L | 12.3 | 22.3 | S. M. L. XL | M. L XL |
| 114 | 12.3 | 22.3 | S M. L. XL | M. L | M. L. XI, | 12.0 | 22.0 | XS. S. M. L. XL | M. L | 12 6 | 226 | S. M. L. XL | M. L XL |
| 175 | 12.5 | 22.5 | S. M. L. XL | M. L. XL | M. L XL | 12.2 | 22.2 | S. M. L. XL | M. L. XL | 12.8 | 22.8 | S. M. L. XL | M L XL |
| 176 | 12.7 | 22.7 | S. M. L. XL | L. XL | L. XL | 12.5 | 22.5 | S. M. L. XL | L. XL | 13.0 | 23.0 | S. M. L. XL | L. XL |
| 177 | 13.0 | 23.0 | S. M. L. XL | L. XL | L. XL | 12.8 | 22.8 | S. M. L. XL | L. XL | 13.2 | 23.2 | S M. L. XL | L. XL |
| 178 | 13.2 | 232 | S. M. L. XL | L. XL | L. XL | 13.1 | 231 | S. M. L. XL | L. XL | 13.5 | 23.5 | S. M. L. XL | L. XL |
| 179 | 13.5 | 23.5 | S. M. L. XL | L. XL | L. XL | 13.3 | 23.3 | S, M, L. XL | L. XL | 13.7 | 23.7 | S. M. L. XL | L. XL |

| | Lower limit | Upper limit | Formula I Formula adapted size I | Recommend | | Lower limit | Upper limit | Formula II Formula adapted size | Recom -mend Male | Lower limit | Upper limit | Formula III Formula adapted size | Recom -mend Female |
|---|---|---|---|---|---|---|---|---|---|---|---|---|---|
| | | | | Male | Female | | | | | | | | |
| 180 | 13.7 | 23.7 | S. M. L. XL | L. XL | L. XL | 13.6 | 23.6 | S. M. L. XL | L. XL | 13.9 | 23.9 | S. M. L. XL | L. XL |
| 181 | 14.0 | 24.0 | S. M. L. XL | L. XL | L. XL | 13.9 | 23.9 | S. M. L XL | L. XL | 141 | 24.1 | M. L XL | L XL |
| 182 | 14.2 | 242 | M. L. XL | L. XL | L. XL | 14.1 | 24.1 | M. L. XL | L. XL | 143 | 24.3 | M. L XL | L. XL |
| 183 | 14.5 | 24.5 | M. L. XL | L. XL | L XL | 14.4 | 24.4 | M. L. XL | L. XL | 146 | 24.6 | M. L XL | L. XL |
| 184 | 147 | 24.7 | M. L. XL | L. XL | L. XL | 14.7 | 247 | M. L. XL | L. XL | 14.8 | 24.8 | M. L XL | L. XL |
| 185 | 15.0 | 25.0 | M. L. XL | L. XL | L. XL | 14.9 | 24.9 | M. L. XL | L. XL | 15.0 | 25.0 | M. L XL | L. XL |
| 186 | 15.2 | 252 | M. L. XL | XL | XL | 15.2 | 25.2 | M. L. XL | XL | 15.2 | 25.2 | M. L XL | XL |
| 187 | 15.5 | 25.5 | M. L. XL | XL | XL | 15.5 | 25.5 | M. L. XL | XL | 15.5 | 25.5 | M. L. XL | XL |
| 188 | 15.7 | 25.7 | M. L. XL | XL | XL | 15.8 | 25.8 | M. L. XL | XL | 15.7 | 25.7 | M. L XL | XL |
| 189 | 16.0 | 26.0 | M. L. XL | XL | XL | 16.0 | 260 | M. L. XL | XL | 15.9 | 25.9 | M. L XL | XL |
| 190 | 16.2 | 26.9 | L. XL | XL | XL | 16.3 | 26.3 | L. XL | XL | 16.1 | 26.1 | L. XL | XL |

About a table 3, as the electrocardiogram measurement shirt of the present invention, it produces those five sizes that XS, S, M, L, and XL, as well as an XLE model that is considered the patient with large body widths, about the actual distance of D as well as a recommended height, a recommend chest circumference, and a recomment waist size ofr each patient, it shows each sex. In the present invention, as shown in the Table 2, it is possible to multiplee select the sizes of the possigble to adjust the size within the allowable size that taken into account the chest circumference, the waist, the weight, or the like.

| Size | D (cm) | Sex | Recommend height(cm) | | Recommend chest circumference (cm) | | Recommend waist(cm) | |
|---|---|---|---|---|---|---|---|---|
| | | | Lower limit | Upper limit | Lower limit | Upper limit | Lower limit | Upper limit |
| XS | 12 | Male | 150 | 160 | 72 | 81 | 58 | 68 |
| | | Female | 147 | 160 | 12 | 81 | 58 | 67 |
| S | 14 | Male | 155 | 165 | 79 | 88 | 68 | 76 |
| | | Female | 150 | 165 | 79 | 88 | 67 | 73 |
| M | 16 | Male | 160 | 175 | 86 | 96 | 76 | 84 |
| | | Female | 160 | 175 | 86 | 96 | 71 | 80 |
| L | 18 | Male | 170 | 185 | 94 | 104 | 84 | 94 |
| | | Female | 170 | 185 | 93 | 102 | 80 | 88 |
| XL | 19 | Male | 175 | 190 | 102 | 112 | 94 | 104 |
| | | Female | 170 | 190 | 100 | 110 | 88 | 97 |
| XLE | 19 | Male | 175 | 190 | 108 | 116 | 100 | 114 |
| | | Female | 170 | 190 | 108 | 116 | 97 | 114 |

Furthermore, it goes without saying that the present invention is not limited to the embodiments described above, and that various modifications can be made without departing from the spirit of the present invention.

**Reference Signs List**

S1 - S51 Steps

1 Clothes

2 Patient

11 Vertice of neckline

12 Center of vertice of neckline

13 Measurement electrode or measurement electrode mounting part position

14 Connector part for fixing measurement device

15 Neutral electrode or neutral electrode mounting part position

16 Stretchable wiring

21 Fifth intercostal assumed height

22 Distance D between first measurement electrode and fifth intercostal assumed height

31 Measuring device

32 Information output part

33 Information input part

34 Recording part

131 First measurement electrode or measurement electrode mounting part position

311 Main control unit

312 Power source

313 AD converter

314 Real time clock

321 Vibration motor

322 LED

323 Speaker

331 Event button

332 Inertial sensor

341 Memory

342 Wireless module

**Claims**

1. An electrocardiographic testing method of the two or more-lead using the clothes comprises at least the three measurement electrodes or a measurement electrode attachment part and a connector part for fixing a measurement device,

   the clothes have at least two or more different sizes,
   the clothes have a neckline encircles the neck, the first measurement electrode or its measurement electrode mounting part is arranged within a circle with a radius of 5cm is centered around a position that 7.5 to 15cm downward from a center point of a straight line connecting to vertices of a left and a right of the neckline, having the following steps:

   (1) a step of obtaining a physique information of a patient;
   (2) a step of selecting a size of clothes according to the physique information of the patient;
   (3) a step of giving the selected clothes and the measuring device to the patient;
   (4) a step of the patient puts on the clothes and the measurement device; and
   (5) a step of starting the electrocardiogram measurement;

   Wherein, the physique information includes a height,
   when the height is H and the vertical distance between the first measurement electrode and the fifth intercostal assumed height is D, H and D satisfy the relationship of the following formula [A], is characterized,

(Math. 1)

$$D = [(H - 119.390) / 2.47] \pm 5.0 \ [A].$$

2. An electrocardiographic testing method of the two or more-lead using the clothes comprises at least the three measurement electrodes or a measurement electrode attachment part and a connector part for fixing a measurement device,

the clothes have at least two or more different sizes,
the clothes have a neckline encircles the neck, the first measurement electrode or its measurement electrode mounting part is arranged within a circle with a radius of 5cm is centered around a position that 7.5 to 15cm downward from a center point of a straight line connecting to vertices of a left and a right of the neckline, having the following steps:

(1) a step of obtaining a physique information of a patient;
(2) a step of selecting a size of clothes according to the physique information of the patient;
(3) a step of giving the selected clothes and the measuring device to the patient;
(4) a step of the patient puts on the clothes and the measurement device; and
(5) a step of starting the electrocardiogram measurement;

wherein, the physique information includes the height,
when the height is H and the vertical distance between the first measurement electrode and the fifth intercostal assumed height is D, H and D satisfy the following formula [B] when the patient is a male, and the following formula [C] when the patient is a female, is characterized,

(Math. 2)

$$D = [(H - 126.388) / 2.23] \pm 5.0 \ [B]$$

(Math. 3)

$$D = [(H - 110.713) / 2.76] \pm 5.0 \ [C].$$

3. The electrocardiographic testing method according to one of claims 1 or 2,
wherein, after the step of (5) is completed, having following a series of steps:

(6) a step of the patient returns the clothes and the device;
(7) a step of reporting the electrocardiogram testing results to the patient;
(8) a step of washing the clothes; and
(9) a step of checking the operation of the clothes and the device, is characterized.

4. The electrocardiographic testing method according to any one of claims 1 to 3,
wherein, the physique information further includes at least one of a body weight, a chest circumference, and an abdominal circumference, is characterized.

5. The electrocardiographic testing method according to any one of claims 1 to 4,
wherein, the lead includes three leads of the lead NASA, the lead CM5, and the lead CC5, is characterized.

6. The electrocardiographic testing method according to any one of claims 1 to 5,
wherein, the step (5) of starting the electrocardiogram measurement includes a step of informing to the patient whether the clothes and the measurement device are correctly put, is characterized.

7. The electrocardiographic testing method according to claim 6,
   wherein, about the step of informing to the patient whether the clothes and the measurement device are correctly put, informing to the patient at least one of a vibration, a light, and a sound from the measurement device or a terminal wirelessly communicating with the measurement device, is characterized.

8. The electrocardiographic testing method according to any one of claims 1 to 7,
   wherein, in the electrocardiographic testing method, a data obtained by the electrocardiogram measurement is transmitted and recorded to a server directly from the measurement device or via the terminal wirelessly communicates with the measurement device, is characterized.

9. The electrocardiographic testing method according to any one of claims 1 to 7,
   wherein, the data obtained by the electrocardiographic measurement is recorded on a recording medium within a device, is characterized.

10. The electrocardiographic testing method according to any one of claims 1 to 9,
    wherein, in the electrocardiographic testing method, the measuring device or the terminal wirelessly communicating with the measuring device includes means for recording a predetermined event, is characterized.

11. The electrocardiographic testing method according to any one of claims 1 to 10,
    wherein, in the electrocardiographic testing method, it is also recorded an exercise data obtained from at least one of an acceleration sensor and a gyro sensor, is characterized.

12. The electrocardiographic testing method according to any one of claims 1 to 11,
    wherein, about a wiring for connecting the measurement electrode or the measurement electrode attachment part and the connector part, when an extension is extended by 20% from the original wiring length, an electrical resistivity increases from 0 to 30%, is characterized.

13. The electrocardiographic testing method according to any one of claims 1 to 12,
    wherein, about a fabric used in a range of 10 cm downward and 5 cm wide from the first measurement electrode or the measurement electrode attachment part, when the vertical direction 4.9N is loaded, an extension rate is 160% or less, is characterized.

14. The electrocardiographic testing method according to any one of claims 1 to 13,
    wherein, about a part of the clothes including a portion for the measurement electrode or the measurement electrode attachment part is attached, it comprises a mechanism can be detached in a whole or in the part from the clothes, is characterized.

Fig. 1

START

S1 — Obtain physical information of patient

S2 — Select clothes size

S3 — Give clothes and device to patient

S4 — Patient puts on clothes and device

S5 — Start electrocardiogram measurement

END

Fig. 2

Fig. 3

```
                    ┌─────────────┐
                    │    START    │
                    └──────┬──────┘
                           │
                           ▼
        ┌──────────────────────────────────────┐
   S1   │  Obtain physical information of       │
        │              patient                  │
        └──────────────────┬───────────────────┘
                           ▼
        ┌──────────────────────────────────────┐
   S2   │          Select clothes size          │
        └──────────────────┬───────────────────┘
                           ▼
        ┌──────────────────────────────────────┐
   S3   │      Give clothes and device to       │
        │              patient                  │
        └──────────────────┬───────────────────┘
                           ▼
        ┌──────────────────────────────────────┐
   S4   │      Patient puts on clothes and      │
        │              device                   │
        └──────────────────┬───────────────────┘
                           ▼
        ┌──────────────────────────────────────┐
   S5   │      Start electrocardiogram          │
        │           measurement                 │
        └──────────────────┬───────────────────┘
                           ▼
        ┌──────────────────────────────────────┐
   S6   │      Patient returns clothes and      │
        │              device                   │
        └──────────────────┬───────────────────┘
                           ▼
        ┌──────────────────────────────────────┐
   S7   │    Report electrocardiogram test      │
        │         results to patient            │
        └──────────────────┬───────────────────┘
                           ▼
        ┌──────────────────────────────────────┐
   S8   │             Wash clothes              │
        └──────────────────┬───────────────────┘
                           ▼
        ┌──────────────────────────────────────┐
   S9   │    Check operation clothes and        │
        │             devices                   │
        └──────────────────┬───────────────────┘
                           ▼
                    ┌─────────────┐
                    │     END     │
                    └─────────────┘
```

22

Fig. 4

Fig. 5

NASA

Fig. 6

CM5

Fig. 7

CC5

Fig. 8

## Fig. 9

```
                    ( START )
                        │
                        ▼
S1    ┌─────────────────────────────┐
      │ Obtain physical information  │
      │        of patient            │
      └─────────────────────────────┘
                        │
                        ▼
S2    ┌─────────────────────────────┐
      │     Select clothes size      │
      └─────────────────────────────┘
                        │
                        ▼
S3    ┌─────────────────────────────┐
      │  Give clothes and device to  │
      │           patient            │
      └─────────────────────────────┘
                        │
                        ▼
S4    ┌─────────────────────────────┐◄──┐
      │  Patient puts on clothes and │   │
      │            device            │   │
      └─────────────────────────────┘   │
Start                   │               │
electrocardiogram       ▼               │
measurement,         ◇                  │
Check putting     ◇     ◇               │
S51             ◇         ◇──────────────┘  NG
                  ◇     ◇
                     ◇
                OK   │
                     ▼
S6    ┌─────────────────────────────┐
      │  Patient returns clothes and │
      │            device            │
      └─────────────────────────────┘
                        │
                        ▼
S7    ┌─────────────────────────────┐
      │ Report electrocardiogram test│
      │      results to patient      │
      └─────────────────────────────┘
                        │
                        ▼
S8    ┌─────────────────────────────┐
      │         Wash clothes         │
      └─────────────────────────────┘
                        │
                        ▼
S9    ┌─────────────────────────────┐
      │  Check operation clothes and │
      │           devices            │
      └─────────────────────────────┘
                        │
                        ▼
                    ( END )
```

Fig. 10

Fig. 11

Fig. 12

## INTERNATIONAL SEARCH REPORT

| International application No. |
| --- |
| **PCT/JP2022/032215** |

**A. CLASSIFICATION OF SUBJECT MATTER**

*A61B 5/256*(2021.01)i; *A61B 5/27*(2021.01)i; *A61B 5/33*(2021.01)i; *A61B 5/332*(2021.01)i
FI:　A61B5/256 210; A61B5/27; A61B5/33 200; A61B5/332

According to International Patent Classification (IPC) or to both national classification and IPC

**B. FIELDS SEARCHED**

Minimum documentation searched (classification system followed by classification symbols)

　　A61B5/256; A61B5/27; A61B5/33; A61B5/332

Documentation searched other than minimum documentation to the extent that such documents are included in the fields searched

　　Published examined utility model applications of Japan 1922-1996
　　Published unexamined utility model applications of Japan 1971-2022
　　Registered utility model specifications of Japan 1996-2022
　　Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2020/111218 A1 (TORAY INDUSTRIES, INC.) 04 June 2020 (2020-06-04) entire text, all drawings | 1-14 |
| A | US 2016/0066809 A1 (LUO, Zhiyuan et al.) 10 March 2016 (2016-03-10) entire text, all drawings | 1-14 |
| A | WO 2019/230921 A1 (JAPANESE ORGANIZATION FOR MEDICAL DEVICE DEVELOPMENT, INC) 05 December 2019 (2019-12-05) entire text, all drawings | 1-14 |
| A | CN 205458657 U (WANG, Yuemeng) 17 August 2016 (2016-08-17) entire text, all drawings | 1-14 |
| A | WO 2018/047814 A1 (TORAY INDUSTRIES, INC.) 15 March 2018 (2018-03-15) entire text, all drawings | 1-14 |
| A | WO 2014/006891 A1 (PANASONIC INTELLECTUAL PROPERTY MANAGEMENT CO., LTD.) 09 January 2014 (2014-01-09) entire text, all drawings | 1-14 |

☑ Further documents are listed in the continuation of Box C. ☑ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **28 October 2022** | **08 November 2022** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

| International application No. |
| --- |
| **PCT/JP2022/032215** |

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2017-518849 A (PEACS B.V.) 13 July 2017 (2017-07-13)<br>entire text, all drawings | 1-14 |
| A | JP 2020-49180 A (MTI LTD) 02 April 2020 (2020-04-02)<br>entire text, all drawings | 1-14 |
| A | JP 2014-517716 A (ZOLL MEDICAL CORPORATION) 24 July 2014 (2014-07-24)<br>entire text, all drawings | 1-14 |
| A | JP 2018-534118 A (AT HEALTH) 22 November 2018 (2018-11-22)<br>entire text, all drawings | 1-14 |
| A | JP 2020-186495 A (TORAY INDUSTRIES, INC.) 19 November 2020 (2020-11-19)<br>entire text, all drawings | 1-14 |
| A | CN 212037517 U (SHIJIAZHUANG YILING PHARMACEUTICAL CO., LTD.) 01<br>December 2020 (2020-12-01)<br>entire text, all drawings | 1-14 |
| A | JP 2019-150255 A (NIPPON KODEN KOGYO KK) 12 September 2019 (2019-09-12)<br>entire text, all drawings | 6-9 |

Form PCT/ISA/210 (second sheet) (January 2015)

**INTERNATIONAL SEARCH REPORT**
Information on patent family members

International application No.

**PCT/JP2022/032215**

| Patent document cited in search report | | | Publication date (day/month/year) | Patent family member(s) | | | Publication date (day/month/year) |
|---|---|---|---|---|---|---|---|
| WO | 2020/111218 | A1 | 04 June 2020 | US 2022/0022797 A1 entire text, all drawings CN 113164125 A | | | |
| US | 2016/0066809 | A1 | 10 March 2016 | (Family: none) | | | |
| WO | 2019/230921 | A1 | 05 December 2019 | US 2021/0236060 A1 entire text, all drawings | | | |
| CN | 205458657 | U | 17 August 2016 | (Family: none) | | | |
| WO | 2018/047814 | A1 | 15 March 2018 | US 2019/0261921 A1 entire text, all drawings CA 3035954 A1 KR 10-2019-0040975 A CN 109688915 A | | | |
| WO | 2014/006891 | A1 | 09 January 2014 | US 2014/0309540 A1 entire text, all drawings | | | |
| JP | 2017-518849 | A | 13 July 2017 | US 2015/0320331 A1 entire text, all drawings WO 2015/170978 A1 CA 2948132 A1 | | | |
| JP | 2020-49180 | A | 02 April 2020 | (Family: none) | | | |
| JP | 2014-517716 | A | 24 July 2014 | US 2012/0293323 A1 entire text, all drawings WO 2012/135059 A2 | | | |
| JP | 2018-534118 | A | 22 November 2018 | US 2018/0333058 A1 entire text, all drawings WO 2017/085403 A1 CA 3005277 A1 CN 108471956 A | | | |
| JP | 2020-186495 | A | 19 November 2020 | (Family: none) | | | |
| CN | 212037517 | U | 01 December 2020 | (Family: none) | | | |
| JP | 2019-150255 | A | 12 September 2019 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020163220 A

**Non-patent literature cited in the description**

- **TAKAMI, M.** *Circulation Reports,* 2021, CR-21
- **FUKUMA, N. et al.** *Scientific reports,* 2019, vol. 9 (1), 1-6
- **NARIHIRO SHIOZAWA.** *Biomedical engineering,* 2016, vol. 54 (3), 135-138
- Electronics Packaging Academic Conference Collection of Lecture Papers 32nd. **JUN HAGIWARA et al.** Electronics Packaging Academic Conference. The Japan Institute of Electronics Packaging, 2018
- **YONGUC, G. N. et al.** Estimation of stature and sex from sternal lengths: an autopsy study. *Anatomical science international,* 2015, vol. 90 (2), 89-96
- **DOWER et al.** Deriving the 12-lead electrocardiogram from four (EASI) electrodes. *Journal of electrocardiology,* 1988, vol. 21, S182-S187
- **FINLAY et al.** Effects of electrode placement errors in the EASI-derived 12-lead electrocardiogram. *Journal of electrocardiology,* 2010, vol. 43 (6), 606-611
- **WINTER et al.** Driven-right-leg circuit design. *IEEE Transactions on Biomedical Engineering,* 1983, vol. 1, 62-66